(19) Europäisches Patentamt
European Patent Office
Office européen des brevets

(11) **EP 3 332 620 B1**

(12) **EUROPÄISCHE PATENTSCHRIFT**

(45) Veröffentlichungstag und Bekanntmachung des Hinweises auf die Patenterteilung:
**01.03.2023   Patentblatt 2023/09**

(21) Anmeldenummer: **16751211.0**

(22) Anmeldetag: **25.07.2016**

(51) Internationale Patentklassifikation (IPC):
**H05H 1/50** $^{(2006.01)}$        **A61L 2/14** $^{(2006.01)}$

(52) Gemeinsame Patentklassifikation (CPC):
**H05H 1/50;** A61L 2/14; A61L 2202/11

(86) Internationale Anmeldenummer:
**PCT/EP2016/067698**

(87) Internationale Veröffentlichungsnummer:
**WO 2017/021194 (09.02.2017 Gazette 2017/06)**

(54) **VORRICHTUNG UND VERFAHREN ZUM ERZEUGEN EINES PLASMAS, SOWIE VERWENDUNG EINER SOLCHEN VORRICHTUNG**

APPARATUS AND METHOD FOR PRODUCING A PLASMA, AND USE OF SUCH AN APPARATUS

DISPOSITIF ET PROCÉDÉ DE GÉNÉRATION D'UN PLASMA, AINSI QU'UTILISATION D'UN DISPOSITIF DE CE GENRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.08.2015   DE 102015215051**

(43) Veröffentlichungstag der Anmeldung:
**13.06.2018   Patentblatt 2018/24**

(73) Patentinhaber: **terraplasma GmbH**
**85748 Garching (DE)**

(72) Erfinder:
• **MORFILL, Gregor**
**81925 München (DE)**
• **LI, Yangfang**
**82140 Olching (DE)**

• **SHIMIZU, Tetsuji**
**Ibaraki 305-0044, (JP)**
• **STEFFES, Bernd**
**82487 Oberammergau (DE)**

(74) Vertreter: **Kordel, Mattias et al**
**Gleiss Große Schrell und Partner mbB**
**Patentanwälte Rechtsanwälte**
**Leitzstrasse 45**
**70469 Stuttgart (DE)**

(56) Entgegenhaltungen:
**EP-A1- 1 993 329        EP-A1- 2 164 309**
**WO-A1-00/65887        WO-A1-2014/035365**
**CN-A- 101 239 269      US-A1- 2007 120 495**
**US-A1- 2008 173 621    US-A1- 2014 180 276**

EP 3 332 620 B1

**Beschreibung**

Die Erfindung betrifft eine Vorrichtung und ein Verfahren zum Erzeugen eines Plasmas sowie eine Verwendung einer solchen Vorrichtung.

Vorrichtungen und Verfahren zum Erzeugen eines Plasmas sind grundsätzlich bekannt (US 2007/0120495 A1). In solchen Vorrichtungen wird typischerweise eine zeitlich und/oder räumlich stationäre Entladung zwischen zwei Elektroden gezündet, wobei durch die Entladung ein Plasma erzeugt wird. Besonders günstig für die Desinfektion oder Entkeimung von Oberflächen oder Fluidströmen und/oder für die Wundbehandlung erweisen sich Vorrichtungen und Verfahren, mittels derer sogenannte kalte, nicht-thermische oder atmosphärische Plasmen erzeugbar sind. Aufgrund der typischerweise vorgesehenen räumlichen Stationarität der Entladung können aber beispielsweise nur vergleichsweise kleine Volumina an Plasma erzeugt oder an Fluiden behandelt werden, oder es bedarf einer Mehrzahl von Entladungen, oder großflächigen Entladungen, welche mit einem entsprechend hohen Einsatz von Energie und/oder elektrischer Leistung verbunden sind und außerdem einen komplizierten apparativen Aufbau erfordern.

Der Erfindung liegt die Aufgabe zugrunde, eine Vorrichtung und ein Verfahren zum Erzeugen eines Plasmas sowie eine Verwendung einer solchen Vorrichtung zu schaffen, wobei die genannten Nachteile nicht auftreten.

Die Aufgabe wird gelöst, indem die Gegenstände der unabhängigen Ansprüche geschaffen werden. Vorteilhafte Ausgestaltungen ergeben sich aus den Unteransprüchen.

Die Aufgabe wird insbesondere gelöst, indem eine Vorrichtung geschaffen wird, die eingerichtet ist zur Erzeugung eines Plasmas, wobei die Vorrichtung wenigstens eine erste Elektrode und wenigstens eine von der ersten Elektrode beabstandet angeordnete zweite Elektrode aufweist. Die Vorrichtung weist weiterhin eine Spannungsquelle auf, die mit wenigstens einer Elektrode, ausgewählt aus der wenigstens einen ersten Elektrode und der wenigstens einen zweiten Elektrode, derart verbunden ist, dass eine Potentialdifferenz zwischen der ersten Elektrode und der zweiten Elektrode durch die Spannungsquelle erzeugbar ist, wobei die erste Elektrode und die zweite Elektrode wenigstens einen Entladungspfad für eine elektrische Entladung in einem Entladungsbereich zwischen der ersten Elektrode und der zweiten Elektrode definieren. Es ist eine Magnetfeldeinrichtung vorgesehen, die eingerichtet und relativ zu der ersten Elektrode und der zweiten Elektrode angeordnet ist, um in dem Entladungsbereich ein Magnetfeld bereitzustellen, sodass ein Magnetfeldvektor des Magnetfelds schräg zu dem Entladungspfad orientiert ist. Auf diese Weise wirkt in dem Entladungsbereich eine Lorentzkraft oder Hall-Kraft auf die Entladung, insbesondere auf ein zwischen den Elektroden ausgebildetes Entladungsfilament, welche dazu führt, dass die Entladung oder das Entladungsfilament räumlich instationär wird, und - getrieben von der Lorentzkraft - in dem Entladungsbereich propagiert. Hierdurch kann mittels der zwischen den Elektroden erzeugten Entladung ein im Vergleich zu einem Volumen der Entladung oder des Entladungsfilaments selbst größerer Bereich überstrichen werden, sodass es möglich ist, mit nur einer Entladung ein vergleichbar großes Volumen zu erfassen und insbesondere ein größeres Plasmavolumen bereitzustellen, und/oder ein größeres Fluidvolumen zu behandeln. Die Vorrichtung ermöglicht es dadurch bei kompaktem und einfachem sowie kostengünstigem Aufbau, vergleichbar größere Plasmavolumina bereitzustellen und/oder vergleichbar größere Fluidvolumina zu beaufschlagen oder zu behandeln.

Die Vorrichtung ist eingerichtet zur Erzeugung eines nicht-thermischen Plasmas. Hierunter wird insbesondere ein Plasma verstanden, bei welchem eine die Verteilung der kinetischen Energie der Elektronen das Plasmas beschreibende Temperatur, die auch als Elektronen-Temperatur bezeichnet wird, nicht identisch und insbesondere sehr viel höher ist als eine die Verteilung der kinetischen Energie der von dem Plasma umfassten Ionen, insbesondere Atomionen oder Molekülionen, beschreibende Temperatur, die auch als Ionen-Temperatur bezeichnet wird. Dabei ist die Elektronen-Temperatur sehr viel höher als die Ionen-Temperatur, wobei die Ionen-Temperatur insbesondere im Bereich von 25 °C bis höchstens 100 °C gewählt werden kann. Ein solches Plasma wird aufgrund der vergleichsweise niedrigen Ionen-Temperatur auch als kaltes Plasma bezeichnet.

Die Vorrichtung ist eingerichtet zur Erzeugung eines solchen nicht-thermischen oder kalten Plasmas vorzugsweise bei Atmosphärendruck, insbesondere also bei ungefähr 1013 mbar, wobei ein solches Plasma auch als atmosphärisches Plasma bezeichnet wird.

Als Plasma wird hier insbesondere ein Materiezustand bezeichnet, bei dem geladene Teilchen mit verschiedennamigen Ladungen in Gasphase nebeneinander vorliegen, wobei sich über ein bestimmtes Volumen gemittelt eine neutrale elektrische Ladung für das betrachtete Volumen ergibt. Das Plasma umfasst außerdem vorzugsweise nichtgeladene Atome und/oder Moleküle, die sich in elektronisch, vibratorisch oder rotatorisch angeregten Zuständen befinden, und die auch als angeregte Teilchen bezeichnet werden, und/oder freie Radikale, insgesamt also insbesondere nichtgeladene, reaktive Atome und/oder Moleküle, die auch als reaktive Teilchen oder reaktive Spezies bezeichnet werden.

Mit dem Begriff "Elektrode" ist hier insbesondere ein elektrisch leitfähiges Element angesprochen, an welches ein Potential angelegt werden kann.

Dass die erste Elektrode und die zweite Elektrode beabstandet voneinander angeordnet sind, bedeutet insbesondere, dass diese räumlich voneinander separiert sind, sodass sie insbesondere auf verschiedene elektrische Potentiale gelegt werden können. Der Abstand zwischen den Elektroden ist also insbesondere so groß gewählt, dass sich diese nicht berühren oder derart beeinflussen, dass sie nur auf ein gemeinsames, gleiches Potential gelegt werden könnten. Dabei ist der Abstand zwischen den beiden Elektroden zugleich zumindest bereichsweise so gewählt, dass eine elektrische Entladung zwischen den Elektroden gezündet werden kann.

Unter einer Spannungsquelle wird hier allgemein eine Einrichtung verstanden, welche geeignet ist, eine Potentialdifferenz zu erzeugen. Dabei ist es möglich, dass die Spannungsquelle nur mit einer der beiden Elektroden, nämlich der ersten Elektrode oder der zweiten Elektrode, verbunden ist, wobei die andere der beiden Elektroden, nämlich die zweite Elektrode oder die erste Elektrode, mit Masse verbunden ist, insbesondere mit einer gleichen Masse, mit welcher auch die Spannungsquelle verbunden ist. Alternativ ist es möglich, dass die Spannungsquelle sowohl mit der ersten Elektrode als auch mit der zweiten Elektrode elektrisch verbunden ist. Es ist möglich, dass die Spannungsquelle eingerichtet ist, um eine der Elektroden auf ein von Masse verschiedenes Potential und die andere der beiden Elektroden auf Masse zu legen, wobei es alternativ oder zusätzlich auch möglich ist, dass die Spannungsquelle eingerichtet ist, um beide Elektroden auf voneinander verschiedene und jeweils von Masse verschiedene Potentiale zu legen. Die Spannungsquelle ist außerdem bevorzugt eingerichtet, um eine bestimmte elektrische Leistung zu erzeugen, sodass eine bestimmte Potentialdifferenz auch bei Stromfluss aufrechterhalten werden kann, wenn nämlich eine Entladung zwischen der ersten Elektrode und der zweiten Elektrode stattfindet.

Unter einem Entladungspfad wird hier insbesondere ein gedachter Entladungspfad verstanden, entlang dem eine Entladung zwischen der ersten Elektrode und der zweiten Elektrode erwartet wird, wenn eine ausreichend hohe Potentialdifferenz zwischen der ersten Elektrode und der zweiten Elektrode erzeugt wird. Der Entladungspfad ist bevorzugt eine kürzeste Verbindung zwischen der ersten Elektrode und der zweiten Elektrode, entlang der die Ausbildung einer elektrischen Entladung bei Anlegen einer ausreichend hohen Potentialdifferenz zwischen der ersten Elektrode und der zweiten Elektrode erwartet wird.

Unter einem Entladungsbereich wird insbesondere ein räumlicher Bereich zwischen der ersten Elektrode und der zweiten Elektrode verstanden, in welchem der wenigstens eine Entladungspfad angeordnet ist, und in welchem vorzugsweise die Entladung - getrieben durch das Magnetfeld - insbesondere entlang wenigstens einer Elektrode propagieren kann.

Dass ein Magnetfeldvektor des Magnetfelds schräg zu dem Entladungspfad orientiert ist, bedeutet insbesondere, dass der Magnetfeldvektor eine Komponente aufweist, die senkrecht auf einer durch den Entladungspfad definierten Richtung steht, oder dass der Magnetfeldvektor vollständig senkrecht zu der durch den Entladungspfad definierten Richtung orientiert ist. Auf diese Weise wirkt eine Lorentz-Kraft auf die entlang des Entladungspfads während der elektrischen Ladung strömenden elektrisch geladenen Teilchen.

Die Spannungsquelle sowie die erste und die zweite Elektrode sind insbesondere so eingerichtet, dass eine Entladung und damit insbesondere ein Entladungsfilament entlang eines Entladungspfads in dem Entladungsbereich zwischen der ersten Elektrode und der zweiten Elektrode erzeugbar ist. Die erste Elektrode, die zweite Elektrode und die Spannungsquelle sind bevorzugt als Plasmaquelle der Vorrichtung ausgebildet.

Vorzugsweise definieren die erste Elektrode und die zweite Elektrode gemeinsam eine Entladungsebene oder Entladungsfläche für den Entladungsbereich, wobei die Entladungsfläche - abhängig vom Verlauf der Elektroden - auch gekrümmt sein kann. Im Betrieb der Vorrichtung erstreckt sich das Entladungsfilament in der Entladungsebene oder Entladungsfläche. Der Magnetfeldvektor ist bevorzugt schräg zu der Entladungsebene oder Entladungsfläche orientiert, bevorzugt steht er senkrecht auf der Entladungsebene oder Entladungsfläche.

Erfindungsgemäß ist vorgesehen, dass die Spannungsquelle als Gleichspannungsquelle ausgebildet ist. Sie ist insbesondere eingerichtet, um ein zeitlich im Wesentlichen konstantes oder konstantes Potential an wenigstens eine der beiden Elektroden, nämlich an die erste Elektrode und/oder an die zweite Elektrode, anzulegen. Die Spannungsquelle kann daher einfach und kostengünstig ausgebildet sein. Insbesondere bedarf es keiner komplexen, hochfrequenten Ansteuerung der Spannungsquelle.

Bevorzugt ist die Spannungsquelle eingerichtet, um eine Potentialdifferenz zwischen der ersten Elektrode und der zweiten Elektrode von mindestens 0,5 kV bis höchstens 9 kV, vorzugsweise von mindestens 1 kV bis höchstens 6 kV, zu erzeugen. Bevorzugt ist die Vorrichtung so eingerichtet, dass eine Durchbruchsspannung zwischen der ersten Elektrode und der zweiten Elektrode höchstens 6 kV, vorzugsweise weniger als 6 kV beträgt, wobei eine Betriebsspannung während einer elektrischen Entladung zwischen der ersten Elektrode und der zweiten Elektrode höchstens 4 kV, vorzugsweise weniger als 4 kV, beträgt.

Die Spannungsquelle ist vorzugsweise eingerichtet, um einen Gleichstrom von mindestens 0,5 mA bis höchstens 20 mA zu liefern, vorzugsweise bei einer Betriebsspannung von höchstens 4 kV, vorzugsweise von weniger als 4 kV, besonders bevorzugt von mindestens 1 kV bis höchstens 4 kV. Die Spannungsquelle weist demnach bevorzugt eine Ausgangsleistung von mindestens 0,5 W bis höchstens 80 W auf.

Gemäß einer Weiterbildung der Erfindung ist vorgesehen, dass die wenigstens eine erste Elektrode als stab- oder drahtförmige Elektrode ausgebildet ist, wobei die wenigstens eine zweite Elektrode als ringförmige Elektrode ausgebildet ist, welche die erste Elektrode - in Umfangsrichtung gesehen - umgreift. Dabei wird eine axiale Richtung definiert durch eine Erstreckung der stab- oder drahtförmigen ersten Elektrode im Bereich der zweiten Elektrode, wobei die Umfangsrichtung die Axialrichtung konzentrisch umgreift. Eine radiale Richtung erstreckt sich dabei senkrecht zu der Axialrichtung.

Der Entladungspfad erstreckt sich bei einer solchen Ausgestaltung in radialer Richtung, wobei der Magnetfeldvektor zumindest eine Komponente in axialer Richtung aufweist, oder sich vollständig in Axialrichtung erstreckt. Entsprechend wirkt eine Lorentzkraft in Umfangsrichtung, sodass die Entladung, insbesondere das Entladungsfilament, in Umfangsrichtung gesehen und somit entlang der ringförmigen, zweiten Elektrode, ringscheibenförmig, vorzugsweise kreisförmig, propagiert. Eine Propagationsgeschwindigkeit der Entladung oder des Entladungsfilaments hängt dabei von der Magnetfeldstärke ab. Effektiv und insbesondere im Zeitmittel wird auf diese Weise quasi ein Plasma-Vorhang oder eine Plasma-Fläche gebildet, welche/welcher den Entladungsbereich durchsetzt. Dieses Phänomen kann als magnetisch organisierter Plasma-Vorhang (magnetically organised plasma sheet - MOPS) bezeichnet werden.

Gemäß einer Weiterbildung der Erfindung ist vorgesehen, dass die wenigstens eine erste Elektrode und die wenigstens eine zweite Elektrode als drahtförmige oder stabförmige Elektrode ausgebildet sind. Dabei erstrecken sie sich vorzugsweise zumindest bereichsweise in einer Ebene oder entlang einer gemeinsamen gekrümmten Fläche, wobei der Entladungsbereich in dieser Ebene oder Fläche angeordnet ist. Dar Magnetfeldvektor steht dann zumindest mit einer Komponente senkrecht auf dieser Ebene oder Fläche, sodass die Entladung oder das Entladungsfilament entlang der Ebene oder Fläche des Entladungsbereichs propagiert. Es entsteht so effektiv, insbesondere im Zeitmittel, ein Plasmavorhang oder eine Plasmafläche in dem Entladungsbereich zwischen den draht- oder stabförmigen Elektroden.

Gemäß einer Weiterbildung der Erfindung ist vorgesehen, dass die wenigstens eine zweite Elektrode als flächige Elektrode ausgebildet ist, welche vorzugsweise eine Mehrzahl von Aussparungen aufweist. Die Aussparungen können eine Vielzahl verschiedener Geometrien aufweisen, beispielsweise eine runde, insbesondere kreisrunde, oder ovale Geometrie, oder eine Geometrie mit zumindest bereichsweise divergierenden und/oder parallelen Wandungen. Die erste Elektrode ist in diesem Fall bevorzugt als draht- oder stabförmige Elektrode ausgebildet und entweder senkrecht zu durch die Aussparungen definierten Flächen orientiert, wobei in diesem Fall bevorzugt in jeder Aussparung eine erste Elektrode - vorzugsweise mittig oder zentral - angeordnet ist; oder die erste Elektrode ist in einer durch die Aussparungen definierten Ebene angeordnet und erstreckt sich entlang der Aussparungen, wobei auch in diesem Fall bevorzugt jeder Aussparung eine erste Elektrode zugeordnet ist, wobei es aber auch möglich ist, dass einer Mehrzahl von Aussparungen, vorzugsweise allen Aussparungen, eine gemeinsame erste Elektrode zugeordnet ist, die beispielsweise in Bereichen zwischen den Aussparungen mit einem Isolatormaterial versehen sein kann, sodass elektrische Entladungen nur im Bereich der Aussparungen stattfinden können. Die erste Elektrode ist dann, wenn sie sich in einer Ebene einer Aussparung erstreckt, bevorzugt benachbart zu einer ersten Wandung der Aussparung und einer zweiten, gegenüberliegenden Wandung der Aussparung gegenüberliegend angeordnet, wobei sie bevorzugt gegenüber der ersten Wandung, zu welcher sie benachbart angeordnet ist, isoliert ist, sodass eine Entladung zwischen der ersten Elektrode und der dieser gegenüberliegenden Wandung der Aussparung erzeugt wird.

Alle hier erläuterten, verschiedenen Ausgestaltungen der Elektroden sind einfach aufgebaut und leicht und kostengünstig herstellbar. Weiterhin weisen sie spezifische Vorteile bei der Erzeugung eines Plasmas insbesondere in Hinblick auf einen in dem Entladungsbereich strömenden Fluidstrom auf.

Ist die wenigstens eine zweite Elektrode als flächige Elektrode mit einer Mehrzahl von Aussparungen ausgebildet, weist der Magnetfeldvektor bevorzugt zumindest eine Komponente auf, welche senkrecht auf einer durch die Aussparungen definierten Ebene oder Fläche steht. Die Entladung propagiert dann getrieben durch die Lorentzkraft jeweils entlang von Berandungen der Aussparungen in einem durch die Aussparungen jeweils definierten Entladungsbereich, der vorzugsweise flächig oder planar ausgebildet sein kann.

Gemäß einer Weiterbildung der Erfindung ist vorgesehen, dass ein Abstand zwischen der ersten Elektrode und der zweiten Elektrode entlang einer Erstreckung wenigstens einer der Elektroden, nämlich der ersten Elektrode und/oder der zweiten Elektrode, konstant ist. Dies ist insbesondere der Fall, wenn die erste Elektrode - beispielsweise als stab- oder drahtförmige Elektrode - im Zentrum oder Mittelpunkt einer ringförmigen zweiten Elektrode angeordnet ist und sich in axialer Richtung erstreckt. Die Entladung läuft dann im Sinne einer Kreisbewegung innerhalb des kreisringförmigen, zwischen der ersten Elektrode und der zweiten Elektrode ausgebildeten Entladungsbereichs um, wobei der Entladungsbereich einen konstanten Radius aufweist. Ein konstanter Abstand zwischen der ersten Elektrode und der zweiten Elektrode kann auch gegeben sein, wenn die erste Elektrode und die zweite Elektrode jeweils als stab- oder drahtförmige Elektrode ausgebildet sind, wobei sie sich dann gemeinsam - stets mit konstantem Abstand voneinander beabstandet - entlang eines bestimmten Pfades, im einfachsten Fall geradlinig, erstrecken. Ein konstanter Abstand ist auch möglich, wenn die zweite Elektrode als flächige Elektrode mit einer Mehrzahl von Aussparungen ausgebildet ist, nämlich entweder in dem Fall, in welchem die Aussparungen eine kreisförmige Berandung aufweisen, wobei die erste Elektrode als draht- oder stabförmige Elektrode jeweils in einem Mittelpunkt einer solchen Aussparung angeordnet ist und sich in axialer Richtung erstreckt, oder wenn die Aussparungen zumindest eine Kante aufweisen, entlang der sich dann - mit konstantem Abstand zu der Kante - auch die erste Elektrode erstreckt. Beispielsweise können in diesem Fall die Aussparungen eine Rechteckform aufweisen. Ein konstanter Abstand hat den Vorteil, dass die Entladung grundsätzlich solange aufrechterhalten werden kann, solange eine die Entladung treibende Potentialdifferenz zwischen den beiden Elektroden durch die Spannungsquelle aufrechterhalten wird. Es kann also insbesondere eine Dauerentladung betrieben werden.

Alternativ oder zusätzlich ist bevorzugt vorgesehen, dass der Abstand zwischen der ersten Elektrode und der zweiten Elektrode entlang einer Erstreckung von wenigstens einer der Elektroden divergiert. Dies ist insbesondere möglich, wenn beide Elektroden als draht- und/oder stabförmige Elektroden ausgebildet sind, wobei sie entlang ihrer Erstreckung auseinanderlaufen können, sodass sich der Abstand zwischen den Elektroden vergrößert, wenn man einer der Elektroden entlang von deren Erstreckung in einer bestimmten Richtung folgt. Eine solche Ausgestaltung kann aber auch realisiert werden, wenn die zweite Elektrode eine Mehrzahl von Aussparungen aufweist, wobei dann wenigstens eine solche Aussparung bevorzugt eine entsprechend geformte Kante aufweist, wobei die erste Elektrode in geeigneter Weise mit divergierendem Abstand an der Aussparung angeordnet sein kann. Ein divergierender Abstand zwischen den Elektroden bietet insbesondere die Möglichkeit, die Entladung in einem bestimmten Abschnitt der Elektroden enden zu lassen, wo nämlich deren Abstand voneinander zu groß wird, als dass die Entladung noch von der Spannungsquelle getrieben werden könnte. Dabei werden die Polarität der beiden Elektroden in Hinblick auf das Magnetfeld und die eine Bewegung der Entladung bewirkende Lorentzkraft bevorzugt so gewählt, dass die Entladung, die vorzugsweise an einem Ort entsteht, wo die beiden Elektroden einen geringsten Abstand zueinander aufweisen, dann in Richtung des zunehmenden Abstands der Elektroden voneinander in dem Entladungsbereich propagiert. Abhängig von den Kennwerten oder eingestellten Parametern der Spannungsquelle erreicht die Entladung dann einen bestimmten Bereich, wo sie zusammenbricht, weil die Spannungsquelle bei dem in diesem Bereich vorliegenden Abstand der Elektroden zueinander die Entladung nicht länger treiben kann. Die Entladung zündet dann vorzugsweise neu an dem Ort, wo die Elektroden ihren geringsten Abstand zueinander aufweisen, und der Vorgang beginnt aufs Neue. Auf diese Weise kann quasi iterativ und fortlaufend eine zyklisch entlang des Beladungsbereichs propagierende Entladung erzeugt werden.

Alternativ oder zusätzlich ist bevorzugt vorgesehen, dass der Abstand zwischen der ersten Elektrode und der zweiten Elektrode in einem ersten Abschnitt konstant ist und in einem zweiten Abschnitt divergiert. Dabei bezieht sich der Begriff "Abschnitt" hier insbesondere auf eine Erstreckung von wenigstens einer der Elektroden, insbesondere auf die Erstreckung beider Elektroden. Eine solche Ausgestaltung ist insbesondere möglich, wenn beide Elektroden als draht- oder stabförmige Elektroden ausgebildet sind, aber auch in einem Fall, in welchem die zweite Elektrode als flächige Elektrode mit einer Mehrzahl von entsprechend geformten Aussparungen ausgebildet ist, wobei dann die erste Elektrode in entsprechender Weise im Bereich der Aussparungen vorgesehen ist. Die Ausgestaltung mit einem abschnittsweise konstanten und abschnittsweise divergierenden Abstand der Elektroden zueinander hat den Vorteil, dass genau abgestimmt werden kann, über welchen Bereich die Entladung propagieren soll, und wo sie dann schließlich aufgrund des zunehmenden Abstands der Elektroden voneinander erlöschen soll.

Der Abstand zwischen den Elektroden beträgt bevorzugt von wenigstens 1 mm bis höchstens 30 mm. Dabei kann er insbesondere einen konstanten Wert in diesem Bereich aufweisen, oder in diesem Bereich divergieren.

Bevorzugt weisen die Elektroden insbesondere im Bereich eines konstanten Abstands oder insgesamt eine Länge auf, die von mindestens 1 mm, vorzugsweise bis höchstens 1 m, beträgt. Über einen solchen Bereich kann ohne weiteres eine Entladung in Luft mit den oben angegebenen Spannungswerten aufrechterhalten und propagiert werden. Es sind prinzipiell aber auch größere Distanzen oder Längen möglich.

Gemäß einer Weiterbildung der Erfindung ist vorgesehen, dass die Magnetfeldeinrichtung wenigstens einen Permanentmagnet aufweist. Gemäß einer bevorzugten Ausgestaltung kann der Permanentmagnet als Ringmagnet ausgebildet sein, wobei er vorzugsweise eine ringförmige zweite Elektrode - in Umfangsrichtung gesehen - umgreift, oder wobei er neben einer Elektrodenanordnung aus der ersten Elektrode und der zweiten Elektrode angeordnet ist, insbesondere wenn die Elektroden stab- oder drahtförmig ausgebildet sind, oder wenn die zweite Elektrode als flächige Elektrode mit einer Mehrzahl von Aussparungen ausgebildet ist. In diesem Fall ist es auch möglich, dass die Magnetfeldeinrichtung eine Mehrzahl von Permanentmagneten aufweist, die auf der flächigen, zweiten Elektrode verteilt, insbesondere den Aussparungen benachbart, angeordnet sind. Jedenfalls ist es möglich, dass die Magnetfeldeinrichtung eine Mehrzahl von Permanentmagneten aufweist. Permanentmagnete haben den Vorteil, dass es keiner elektrischen Ansteuerung der Magnetfeldeinrichtung bedarf, sodass die Vorrichtung einfach und mit vergleichsweise geringer elektrischer Leistungsaufnahme ausgebildet sein kann.

Alternativ oder zusätzlich ist es möglich, dass die Magnetfeldeinrichtung wenigstens einen Elektromagnet aufweist. Vorteilhaft an einem Elektromagnet ist, dass die Magnetfeldeinrichtung in definierter Weise an- und abgeschaltet werden kann, wobei auch eine einfache Variation der Feldstärke der Magnetfeldeinrichtung möglich ist. Weiterhin hängt die Feldstärke der Magnetfeldeinrichtung im Wesentlichen von einem Strom durch den Elektromagnet ab und unterliegt nicht einer Schwächung durch Alterungseffekte, wie dies bei einem Permanentmagnet der Fall sein kann.

Gemäß einer Weiterbildung der Erfindung ist vorgesehen, dass die Magnetfeldeinrichtung wenigstens einen Ringmagnet aufweist. Dieser kann insbesondere die Anordnung aus der wenigstens einen ersten Elektrode und der wenigstens einen zweiten Elektrode - in Umfangsrichtung gesehen - umgreifen, oder neben wenigstens einer der Elektroden, vorzugsweise neben beiden Elektroden, also der der Elektrodenanordnung, angeordnet sein.

Alternativ oder zusätzlich ist es möglich, dass die Magnetfeldeinrichtung wenigstens eine elektrische Spule aufweist. Auch diese kann - analog zu dem Ringmagnet - wenigstens eine der Elektroden, vorzugsweise beide Elektroden - in Umfangsrichtung umgreifen, oder neben wenigstens einer der Elektroden, vorzugsweise neben beiden Elektroden, angeordnet sein.

Es ist möglich, dass die Magnetfeldeinrichtung eine Mehrzahl von Ringmagneten und/oder eine Mehrzahl von Spulen aufweist.

Unter einer Spule wird insbesondere eine elektrische Induktionsspule verstanden, die in ihrer einfachsten Ausführungsform einen gewendelten elektrischen Leiter aufweisen oder aus einem gewendelten elektrischen Leiter bestehen kann.

Alternativ oder zusätzlich weist die Magnetfeldeinrichtung bevorzugt eine Mehrzahl von Stabmagneten und/oder Spulen auf, welche entlang zumindest einer Elektrode, ausgewählt aus der wenigstens einen ersten Elektrode und/oder der wenigstens einen zweiten Elektrode, angeordnet ist. Dabei ist es möglich, dass eine Mehrzahl von Stabmagneten oder Spulen um eine ringförmige zweite Elektrode und/oder um wenigstens eine Aussparung einer flächigen zweiten Elektrode angeordnet sind. Eine Nordpol-Südpol-Orientierung der Stabmagnete und/oder eine Längsachse der Spulen ist dabei vorzugsweise parallel zu einer Längsachse der ringförmigen zweiten Elektrode oder senkrecht zu der flächigen zweiten Elektrode orientiert. Es ist auch möglich, dass eine Mehrzahl von Stabmagneten und/oder Spulen entlang einer draht- oder stabförmigen Elektrode angeordnet ist. Dabei ist die Nordpol/Südpol-Orientierung der Stabmagnete und/oder die Längsachse der Spulen insbesondere senkrecht zu der Erstreckung der Elektrode angeordnet.

Eine Ausgestaltung mit einer Mehrzahl von Stabmagneten und/oder Spulen ist gegebenenfalls einfacher zu realisieren, als eine Ausgestaltung mit einem Ringmagneten oder nur einer Spule.

Alternativ oder zusätzlich ist bevorzugt vorgesehen, dass die Magnetfeldeinrichtung wenigstens einen Bandmagneten aufweist, welcher sich entlang zumindest einer der Elektroden erstreckt. Diese Ausgestaltung ist besonders vorteilhaft in Zusammenhang mit einer Elektrodenanordnung aus wenigstens zwei stab- oder drahtförmigen Elektroden, wobei ein solcher Bandmagnet entlang der Erstreckung wenigstens einer dieser Elektroden ohne weiteres angeordnet werden kann. Auch eine Anordnung eines solchen Bandmagneten entlang wenigstens einer Aussparung einer flächigen Elektrode ist ohne weiteres möglich.

Unter einem Bandmagnet wird hier insbesondere ein magnetisches Element verstanden, welches sich entlang einer ersten Richtung erstreckt, wobei die Erstreckung entlang der ersten Richtung größer ist als eine Breite des Elements in einer zweiten Richtung und eine Höhe des Elements in einer dritten Richtung, wobei die erste Richtung, die zweite Richtung und die dritte Richtung aufeinander senkrecht stehen. Ein solcher Bandmagnet kann beispielsweise ungefähr 3 mm breit, zwischen 1 cm und 2 cm hoch, und bis zu 20 cm lang sein. Ein solcher Bandmagnet stellt quasi eine integrale, einstückige Hintereinander-Anordnung einer Mehrzahl von Magnetelementen dar, und ist beispielsweise im Wege eines Sinterprozesses aus einem magnetischen Material herstellbar.

Ein solcher Bandmagnet kann insbesondere entlang einer Anordnung von draht- oder stabförmigen Elektroden, oder entlang von wenigstens einer Kante einer Aussparung in einer flächigen Elektrode angeordnet werden und stellt dort eine strukturell einfachere und zugleich vom Magnetfeldverlauf her günstigere Ausgestaltung dar, als eine Nebeneinanderreihung separater Stabmagnete.

Erfindungsgemäß ist vorgesehen, dass die Vorrichtung einen Leitungsabschnitt aufweist, der mit dem Entladungsbereich in Fluidverbindung ist, sodass ein Fluid entlang des Leitungsabschnitts durch den Entladungsbereich geleitet werden kann. Auf diese Weise ist es möglich, ein Fluid mit dem in dem Entladungsbereich gebildeten Plasma zu behandeln und beispielsweise zu desinfizieren oder zu sterilisieren, üble Gerüche zu entfernen, Allergene zu entfernen, das Fluid mikrobiell zu inaktivieren, oder Partikel zu entfernen. Dabei umfasst der Entladungsbereich, in welchem die Entladung propagiert, bevorzugt einen gesamten lichten Durchmesser der Vorrichtung am Ort des Entladungsbereichs, sodass das Fluid vollständig durch den Entladungsbereich geführt wird. Es kann auf diese Weise sichergestellt werden, dass das gesamte, den Entladungsbereich durchsetzende Fluid von der Entladung und damit auch dem Plasma beaufschlagt wird.

Die Aufgabe wird auch gelöst, indem ein Verfahren zum Erzeugen eines Plasmas geschaffen wird, welches folgende Schritte aufweist: Es wird wenigstens ein Entladungsfilament entlang eines Entladungspfads in einem Entladungsbereich zwischen wenigstens zwei Elektroden erzeugt, wobei ein Magnetfeld bereitgestellt wird, welches den Entladungsbereich schräg zu dem Entladungspfad, bevorzugt senkrecht zu dem Entladungspfad, durchsetzt. Das Entladungsfilament wird dabei durch das Magnetfeld zu einer propagierenden Bewegung in dem Entladungsbereich angetrieben. Das Verfahren wird durchgeführt mittels einer Vorrichtung nach einem der zuvor beschriebenen Ausführungsbeispiele. Es ergeben sich insbesondere die Vorteile, die bereits in Zusammenhang mit der Vorrichtung erläutert wurden. Unter einem Entladungsfilament wird insbesondere ein Strom von geladenen Teilchen verstanden, welcher entlang eines Potentialgradienten fließt. Dabei entsteht eine elektrische Entladung zwischen zwei Elektroden typischerweise, indem zunächst ein Entladungskanal, insbesondere ein sogenannter Streamer, insbesondere durch Ionisation von Teilchen entlang des Entladungskanals, gebildet wird, bevor die Bewegung geladener Teilchen entlang des Entladungskanals einsetzt. Geladene Teilchen strömen dann während der Entladung entlang des gebildeten Entladungskanals und bilden so ein Entladungsfilament aus. Die geladenen Teilchen erfahren in dem Magnetfeld eine Lorentzkraft, welche auf jedes einzelne entlang des Entladungskanals propagierende Teilchen und somit quasi insgesamt auf das Entladungsfilament wirkt, sodass das Entladungsfilament schließlich insgesamt entlang des Entladungsbereichs propagiert.

Erfindungsgemäß ist vorgesehen, dass ein Fluid durch den Entladungsbereich geleitet wird. Dabei kann das Fluid in vorteilhafter Weise mit dem Entladungsfilament und insbesondere mit dem Plasma, welches durch die Entladung gebildet wird, beaufschlagt werden.

Die propagierende Bewegung des Entladungsfilaments und eine Strömungsgeschwindigkeit des Fluids durch den Entladungsbereich werden so aufeinander abgestimmt, dass jedes Volumenelement des den Entladungsbereich durchströmenden Fluids wenigstens einmal von dem Entladungsfilament überstrichen wird. Es ist insoweit festzustellen, dass bevorzugt eine periodische Bewegung des Entladungsfilaments in dem Entladungsbereich erzeugt wird, nämlich beispielsweise gemäß einer ersten Ausgestaltung eine periodische Kreisbewegung, oder allgemein eine zyklische Bewegung, und/oder gemäß einer zweiten Ausgestaltung eine periodische Bewegung dergestalt, dass die Entladung an einem ersten Ort entsteht, dann entlang einer bestimmten Strecke propagiert, und an einem zweiten Ort - beispielsweise durch einen zunehmenden Abstand der Elektroden voneinander - erlischt. Die Entladung startet dann an dem ersten Ort neu, wobei sich der gesamte Vorgang zyklisch mit einer bestimmten Frequenz wiederholt.

Unter einem Volumenelement des strömenden Fluids wird hier ein gedachtes Volumen verstanden, welches sich in axialer Richtung, also senkrecht zu dem Entladungspfad und insbesondere in Richtung des Magnetfeldvektors, durch den Entladungsbereich bewegt und die Dicke - in axialer Richtung gemessen - des Entladungsfilaments aufweist.

Um sicherzustellen, dass jedes Volumenelement des den Entladungsbereich durchströmenden Fluids wenigstens einmal von der Entladung überstrichen wird, wird nun diese Frequenz des in dem Entladungsbereich propagierenden Entladungsfilaments auf die Strömungsgeschwindigkeit des Fluids in geeigneter Weise abgestimmt.

Beispielsweise ergibt sich für eine Anordnung einer ringförmigen zweiten Elektrode mit einer stab- oder drahtförmigen ersten Elektrode in deren Zentrum, insbesondere also eine kreisringförmige Entladung mit einer - in axialer Richtung gemessenen - Dicke d und einer Strömungsgeschwindigkeit des Fluids in axialer Richtung mit einer Geschwindigkeit v, das die Umlauf- oder Rotationsfrequenz der Entladung $\Omega$ größer sein muss als 2ITv/d, damit die Entladung aus Sicht des strömenden Fluids quasi wie ein Plasmavorhang erscheint, sodass also jedes Volumenelement des strömenden Fluids zumindest einmal von dem Entladungsfilament überstrichen wird. Beispielsweise ergibt sich konkret für eine in axialer Richtung gemessene Dicke der Entladung d von 1 mm und einer Strömungsgeschwindigkeit von 1 m/s für das Fluid, dass die Rotationsfrequenz der Entladung größer sein muss als 6000 s$^{-1}$ (als Kreisfrequenz berechnet), oder - unter Berücksichtigung des Faktors $2\pi$ - eine Frequenz von mehr als 1000 s$^{-1}$ aufweisen muss.

Die Rotationsgeschwindigkeit der Entladung wird durch die Lorentzkraft bestimmt und ergibt sich beispielsweise nach folgender Gleichung

$$ v_\varphi = v_r \left( \frac{\omega_e}{v_{en}} \right) . \qquad\qquad (1) $$

wobei $v_\varphi$ = $r\Omega$ = $2\pi r f$ die azimutale (Umfangs-)Rotationsgeschwindigkeit in einem Abstand r vom Mittelpunkt der zylindrischen Anordnung der beiden Elektroden 3, 5 ist, wobei v, die radiale Ausbreitungsgeschwindigkeit der Entladung ist, die ohne weiteres aus den Parametern der Spannungsquelle und vorzugsweise dem Elektrodenabstand berechnet werden kann, wobei weiter $\omega_e$ die Elektronen-Gyrofrequenz in dem Magnetfeld B ist, und worin $v_{en}$ die Kollisionsrate mit atmosphärischen Neutralteilchen als dominierender Bremsmechanismus ist.

Anhand der Gleichung kann ohne weiteres ermittelt werden, dass Rotationsfrequenzen, die für eine effiziente Behandlung eines strömenden Fluids mit beispielsweise einer Strömungsgeschwindigkeit von 1 m/s bereitgestellt werden müssen, durch ein axiales Magnetfeld, welches größer ist als 0,01 T, vorzugsweise größer als 0,1 T, verwirklicht werden können. Besonders bevorzugt werden allerdings Magnetfeldstärken von mindestens 0,1 T bis höchstens 1 T verwendet. Dabei ist insbesondere für die Obergrenze derzeit noch die Art der verfügbaren Magnete für die Magnetfeldeinrichtung limitierend. Selbstverständlich sind höhere Feldstärken wünschenswert und werden bevorzugt eingesetzt, soweit entsprechende Magnetfeldeinrichtungen zur Verfügung stehen. Derzeit ist es jedoch ohne weiteres möglich, insbesondere mit kleinen, vorzugsweise zylindrischen Neodym-Permanentmagneten entsprechende Feldstärken im Bereich von mindestens 0,1 T bis höchstens 1 T bereitzustellen. Es wird eine Ausführungsform des Verfahrens bevorzugt, die sich dadurch auszeichnet, dass der Entladungsbereich unter Atmosphärendruck gehalten wird, vorzugsweise also bei 1013 mbar oder bei einem lokal herrschenden Umgebungsdruck. Das Verfahren ist auf diese Weise besonders kostengünstig und einfach durchführbar, weil es weder einer Einrichtung zur Evakuierung noch einer Einrichtung bedarf, welche eingerichtet wäre, um den Entladungsbereich unter höheren Druck zu setzen. Dabei ist es mithilfe der hier vorgeschlagenen Vorrichtung ohne weiteres möglich, eine Plasmaentladung unter Atmosphärendruck zu erzeugen. Selbstverständlich ist aber mittels der Vorrichtung und im Rahmen des Verfahrens auch ein

Arbeiten unter anderen Drücken, insbesondere unter höheren oder niedrigeren Drücken möglich. Gemäß der Erfindung ist vorgesehen, dass durch den Entladungsbereich ein Fluid geführt wird, welches vorzugsweise ausgewählt ist aus einer Gruppe bestehend aus Luft, Stickstoff. einem Edelgas, insbesondere Argon, Kohlendioxid, einem Gasgemisch, und einem Gemisch aus wenigstens einem Gas und wenigstens einer Flüssigkeit, insbesondere Wasserdampf. Dabei beziehen sich die Begriffe "Gas" und "Flüssigkeit" insbesondere auf Normalbedingungen, bezeichnen also einen Aggregatzustand eines Stoffs insbesondere bei 25 °C oder 298 K und 1013 mbar. Die hier vorgeschlagene Vorrichtung und das hier vorgeschlagene Verfahren können insbesondere genutzt werden, um Fluide, insbesondere ausgewählt aus der zuvor genannten Gruppe, zu aktivieren oder zu reinigen.

Gemäß einer Weiterbildung der Erfindung ist vorgesehen, dass das den Entladungsbereich durchströmende Fluid gereinigt wird von a) Gerüchen, b) Allergenen, c) Mikroorganismen, insbesondere Mikroben, Bakterien, Sporen, Pilzen, und Viren, und/oder d) von Partikeln. In dieser Weise können sehr vorteilhaft verschiedenste Fluide von verschiedensten Belastungen gereinigt werden, sodass die Vorrichtung und das Verfahren vielseitig einsetzbar sind.

Die Aufgabe wird schließlich auch gelöst, indem eine Verwendung einer Vorrichtung nach einem der zuvor beschriebenen Ausführungsbeispiele als Luftreinigungseinrichtung geschaffen wird. Dabei wird die Vorrichtung als Luftreinigungseinrichtung insbesondere verwendet in einer Wohnung, beispielsweise als Table-Top-Vorrichtung oder als Teil einer Belüftungs- und/oder Klimaanlage, in einer Küche, insbesondere in einer Küche eines Gastronomiebetriebs, oder in einer Großküche, in einem Stall oder einer Fleischproduktionseinrichtung, in einer Chemieeinrichtung, in einem Büro, in einer Fabrik, in einer Kläranlage, in einer Mülldeponie, und/oder in einer medizinischen Einrichtung, beispielsweise einer Arztpraxis, einer Krankenstation oder Pflegestation, oder einem Krankenhaus. In all diesen Bereichen kann die Vorrichtung sinnvoll, zielführend und vorteilhaft verwendet werden, um Luft von Belastungen, insbesondere der oben genannten Art, zu reinigen.

Unter einer Chemieeinrichtung wird dabei insbesondere eine Produktionsstätte für wenigstens eine Chemikalie, eine Raffinerie, eine Produktionsstätte für wenigstens ein pharmazeutisches Produkt, eine Forschungseinrichtung, eine Laboreinrichtung, auch eine Analyselaboreinrichtung, oder eine andere Einrichtung verstanden, in der chemische Experimente, Versuche und/oder Reaktionen durchgeführt werden.

Alternativ oder zusätzlich wird eine Verwendung der Vorrichtung zur Reinigung eines Fluids von Gerüchen, Allergenen, Mikroorganismen, insbesondere Mikroben, Bakterien, Sporen, Pilzen, und/oder Viren, und/oder von Partikeln, insbesondere durch elektronische Dissoziation (electron impact dissociation), vorgeschlagen. Insbesondere zu diesem Zweck kann die Vorrichtung in vorteilhafter Weise eingesetzt werden.

Die Beschreibung der Vorrichtung einerseits sowie des Verfahrens und der Verwendung andererseits sind komplementär zueinander zu verstehen. Merkmale der Vorrichtung, die explizit oder implizit in Zusammenhang mit dem Verfahren oder der Verwendung beschrieben wurden, sind bevorzugt einzeln oder miteinander kombiniert Merkmale eines bevorzugten Ausführungsbeispiels der Vorrichtung. Verfahrensschritte des Verfahrens oder Aspekte der Verwendung, die explizit oder implizit in Zusammenhang mit der Vorrichtung beschrieben wurden, sind bevorzugt einzeln oder miteinander kombiniert Schritte oder Aspekte einer bevorzugten Ausführungsform des Verfahrens oder der Verwendung. Das Verfahren und/oder die Verwendung zeichnet/zeichnen sich bevorzugt durch wenigstens einen Verfahrensschritt oder einen Aspekt aus, der durch wenigstens ein Merkmal eines erfindungsgemäßen oder bevorzugten Ausführungsbeispiels der Vorrichtung bedingt ist. Die Vorrichtung zeichnet sich bevorzugt durch wenigstens ein Merkmal aus, welches durch wenigstens einen Schritt oder Aspekt einer erfindungsgemäßen oder bevorzugten Ausführungsform des Verfahrens oder der Verwendung bedingt ist.

Die Erfindung wird im Folgenden anhand der Zeichnung näher erläutert. Dabei zeigen:

Figur 1    eine schematische Darstellung eines ersten Ausführungsbeispiels einer Vorrichtung;

Figur 2    eine schematische Darstellung eines zweiten Ausführungsbeispiels der Vorrichtung;

Figur 3    eine schematische Darstellung eines dritten Ausführungsbeispiels der Vorrichtung;

Figur 4    eine schematische Darstellung eines vierten Ausführungsbeispiels der Vorrichtung;

Figur 5    eine schematische Darstellung eines fünften Ausführungsbeispiels der Vorrichtung; und

Figur 6    eine schematische Darstellung eines sechsten Ausführungsbeispiels der Vorrichtung.

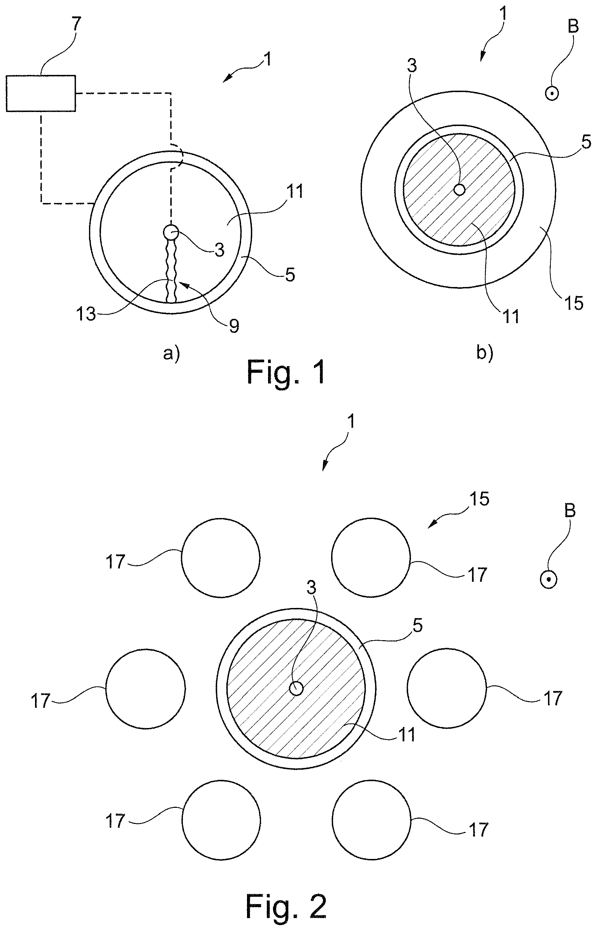

**Fig. 1** zeigt eine schematische Darstellung eines ersten Ausführungsbeispiels einer Vorrichtung 1 zur Erzeugung eines Plasmas. Dabei ist in Figur 1a) eine Vorrichtung 1 dargestellt, die eine erste Elektrode 3 und eine zweite Elektrode 5 aufweist, wobei hier die erste Elektrode 3 als stabförmige oder drahtförmige Elektrode ausgebildet ist, wobei die zweite Elektrode 5 als Ringelektrode ausgebildet ist, welche die erste Elektrode 3 - in Umfangsrichtung gesehen - umgreift. Eine axiale Richtung erstreckt sich dabei senkrecht zur Bildebene von Figur 1a), wobei eine radiale Richtung senkrecht auf der axialen Richtung steht und in der Bildebene angeordnet ist, und wobei sich eine Umfangsrichtung konzentrisch um die axiale Richtung erstreckt. Die erste Elektrode 3 und die zweite Elektrode 5 sind voneinander beabstandet angeordnet.

Insbesondere ist die zweite Elektrode 5 hier kreisringförmig ausgebildet, wobei die erste Elektrode 3 im Mittelpunkt des durch die zweite Elektrode 5 definierten Kreises angeordnet ist. Schematisch ist eine Spannungsquelle 7 angedeutet, welche die Vorrichtung 1 aufweist, und welche mit der ersten Elektrode 3 und der zweiten Elektrode 5 derart verbunden ist, dass eine Potentialdifferenz zwischen der ersten Elektrode 3 und der zweiten Elektrode 5 durch die Spannungsquelle 7 erzeugbar ist. Die erste Elektrode 3 und die zweite Elektrode 5 definieren einen Entladungspfad 9 für eine Entladung in einem Entladungsbereich 11 zwischen der ersten Elektrode 3 und der zweiten Elektrode 5. Dabei zeigt sich, dass hier aufgrund des zylindersymmetrischen Aufbaus der Anordnung aus der ersten Elektrode 3 und der zweiten Elektrode 5 eine ganze Schar von Entladungspfaden 9 definiert wird, die sich - insbesondere in radialer Richtung - entlang des Umfangs des kreisringförmigen Entladungsbereichs 11 erstrecken, wobei aufgrund des hier konstanten Abstands zwischen der ersten Elektrode 3 und der zweiten Elektrode 5 nicht sicher vorhergesagt werden kann, an welcher Stelle eine Entladung tatsächlich initial stattfinden wird, welcher Entladungspfad 9 aus der Schar möglicher Entladungspfade also initial realisiert werden wird.

Die Spannungsquelle 7 ist erfindungsgemäß als Gleichspannungsquelle ausgebildet, wobei sie insbesondere eingerichtet ist, um eine Potentialdifferenz zwischen der ersten Elektrode 3 und der zweiten Elektrode 5 von mindestens 0,5 kV bis höchstens 9 kV, vorzugsweise von mindestens 1 kV bis höchstens 6 kV, zu erzeugen, wobei die Spannungsquelle weiter bevorzugt eingerichtet ist, um eine Stromstärke von mindestens 0,5 mA bis höchstens 20 mA zu liefern.

In Figur 1a) ist noch keine Magnetfeldeinrichtung dargestellt, um die Funktionsweise der Vorrichtung 1 schrittweise näher zu erläutern. Wird ohne Wirkung eines Magnetfelds - beispielsweise bei ausgeschalteter oder entfernter Magnetfeldeinrichtung - eine Entladung zwischen der ersten Elektrode 3 und der zweiten Elektrode 5 gezündet, entsteht ein hier schematisch angedeutetes Entladungsfilament 13, welches sich - bis auf gegebenenfalls Störungen durch beispielsweise Luft- oder Gasbewegungen - in radialer Richtung in dem Entladungsbereich 11 erstreckt. Dabei ist dieses Entladungsfilament 13 ohne Wirkung eines magnetischen Feldes in dem Sinne stationär, dass der einmal gebildete Entladungspfad 9 zeitlich konstant erhalten bleibt.

Figur 1b) zeigt die Vorrichtung 1 ergänzt um eine Magnetfeldeinrichtung 15, die eingerichtet und so relativ zu der ersten Elektrode 3 und der zweiten Elektrode 5 angeordnet ist, dass sie ein Magnetfeld in dem Entladungsbereich 11 bereitstellt, sodass ein Magnetfeldvektor B des Magnetfelds schräg, vorzugsweise senkrecht, zu dem Entladungspfad 9 orientiert ist. Es zeigt sich hier, dass der Magnetfeldvektor B senkrecht auf der Bildebene von Figur 1 steht. Die Magnetfeldeinrichtung 15 ist bei dem hier konkret dargestellten Ausführungsbeispiel der Vorrichtung 1 vorzugsweise als Permanentmagnet, und insbesondere als Ringmagnet ausgebildet, welcher die zweite Elektrode 5 und die erste Elektrode 3 - in Umfangsrichtung gesehen - umgreift. Alternativ ist es aber auch möglich, dass die Magnetfeldeinrichtung 15 einen entsprechend angeordneten Elektromagnet, insbesondere eine elektrische Spule aufweist.

Die Spannungsquelle 7 ist in Figur 1b) aus Gründen der vereinfachten Darstellung nicht dargestellt, gleichwohl jedoch vorhanden.

Wird nun eine Entladung zwischen der ersten Elektrode 3 und der zweiten Elektrode 5 gezündet, wirkt auf das Entladungsfilament 13 eine Lorentzkraft, sodass das Entladungsfilament 13 in Rotation versetzt wird und mit einer bestimmten Rotationsfrequenz entlang des kreisringförmigen Entladungsbereichs 11 propagiert, wobei die Rotationsfrequenz insbesondere von der Feldstärke des Magnetfelds abhängt. Das Entladungsfilament 13 überstreicht also den gesamten Entladungsbereich 11 und bildet in diesem - insbesondere im Zeitmittel gesehen - einen Art Plasma-Vorhang, wobei der gesamte Entladungsbereich 11 mit Plasma beaufschlagt wird.

**Fig. 2** zeigt eine schematische Darstellung eines zweiten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Die Magnetfeldeinrichtung 15 umfasst bei diesem Ausführungsbeispiel eine Mehrzahl von Stabmagneten 17, die - vorzugsweise mit gleichen Winkelabständen, also symmetrisch - in Umfangsrichtung gesehen um die erste Elektrode 3 und die zweite Elektrode 5 herum angeordnet sind. Die Stabmagnete 17 sind dabei so orientiert, dass auch hier der Magnetfeldvektor B senkrecht auf der Bildebene von Figur 2 steht. Alternativ zu den Stabmagneten 17 ist es auch möglich, dass die Magnetfeldeinrichtung 15 eine Mehrzahl von Elektromagneten, insbesondere Spulen aufweist, welche entsprechend um die Anordnung aus den Elektroden 3, 5 herum angeordnet sind. Besonders bevorzugt weist die Vorrichtung 1 und insbesondere die Magnetfeldeinrichtung 15 sechs Stabmagnete 17 auf.

Eine Anordnung mit Stabmagneten 17 oder räumlich verteilt angeordneten Spulen ist gegebenenfalls einfacher und kostengünstiger herstellbar, als eine Magnetfeldeinrichtung 15 mit einem größeren Ringmagnet oder einer entsprechend größeren Spule, welche die Anordnung der Elektroden 3, 5 umgreift, wie dies in Figur 1b) dargestellt ist.

Der sich physikalisch ergebende Effekt in Hinblick auf die Rotation des Entladungsfilaments 13 entlang des Entladungsbereichs 11 ist allerdings bei dem ersten Ausführungsbeispiel gemäß Figur 1b) und dem zweiten Ausführungsbeispiel gemäß Figur 2 derselbe. Es ist lediglich zu beachten, dass typischerweise das Magnetfeld, welches durch die Mehrzahl von Stabmagneten 17 oder eine Mehrzahl von Spulen erzeugt wird, inhomogener ist als das durch einen ringförmigen Magnet oder eine einzige Spule erzeugte Magnetfeld im Fall des ersten Ausführungsbeispiels. Hierdurch ist es bei dem zweiten Ausführungsbeispiel gemäß Figur 2 möglich, dass das Entladungsfilament 13 entlang einer Umfangslinie in dem Entladungsbereich 11 variierende Umlaufgeschwindigkeiten aufweist, weil auch die Lorentzkraft lokal variiert.

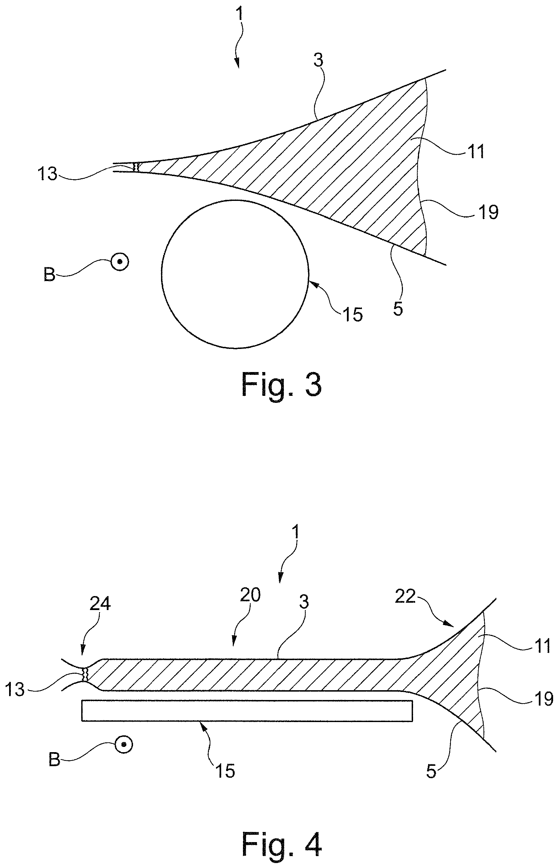

**Fig. 3** zeigt eine schematische Darstellung eines dritten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Die erste Elektrode 3 und die zweite Elektrode 5 sind bei diesem Ausführungsbeispiel beide als stab- oder insbesondere drahtförmige Elektroden ausgebildet, wobei sie einen divergierenden Abstand zueinander aufweisen. Die Magnetfeldeinrichtung 15, welche hier bevorzugt einen Permanentmagnet, insbesondere einen Ring- oder Stabmagnet, aufweist, ist hier neben der Anordnung aus den Elektroden 3, 5 angeordnet, und zwar derart, dass auch hier der Magnetfeldvektor B senkrecht auf der Bildebene zu Figur 3 steht. Alternativ ist es möglich, dass die Magnetfeldeinrichtung 15 einen Elektromagneten oder wenigstens eine Spule aufweist. Wird eine Potentialdifferenz an die Elektroden 3, 5 angelegt, ergibt sich ein Entladungsfilament 13 insbesondere am Ort eines kürzesten Abstands zwischen den Elektroden 3, 5, wobei durch das Magnetfeld eine Lorentzkraft auf das Entladungsfilament 13 wirkt, sodass dieses entlang der Erstreckung der Elektroden 3, 5 - hier nach rechts - wandert und den Entladungsbereich 11 überstreicht, bis ein Abstand zwischen den Elektroden 3, 5 in einem Abbruchbereich 19 zu groß wird, sodass die Spannungsquelle 7 die Entladung nicht mehr treiben kann. Die konkrete Lage des Abbuchbereichs 19 hängt insbesondere von der Spannung, mithin der Potentialdifferenz, zwischen den Elektroden 3, 5 ab. Nach Zusammenbruch der Entladung startet diese neu am Ort des geringsten Abstands zwischen den Elektroden 3, 5, und der zuvor beschriebene Prozess wiederholt sich zyklisch. Auch dies führt - insbesondere im Zeitmittel - quasi zur Ausbildung eines Plasma-Vorhangs in dem Entladungsbereich 11, der zyklisch mit einer bestimmten Frequenz von dem Entladungsfilament 13 überstrichen wird. Dabei hängt diese Frequenz insbesondere von der Feldstärke des Magnetfelds und der Länge der Elektroden 3, 5 zwischen dem Entstehungsort des Entladungsfilaments 13 und dem Abbruchbereich 19 ab.

Der Abstand zwischen den Elektroden 3, 5 beträgt bevorzugt zwischen mindestens 1 mm und höchstens 30 mm in dem Entladungsbereich 11.

Der Abstand zwischen den Elektroden 3, 5 in dem Abbruchbereich 19 kann beispielsweise 3 cm betragen.

**Fig. 4** zeigt eine schematische Darstellung eines vierten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Auch bei diesem Ausführungsbeispiel sind die Elektroden 3, 5 als stab- oder insbesondere drahtförmige Elektroden ausgebildet, wobei ein Abstand zwischen den Elektroden 3, 5 abschnittsweise konstant ist und abschnittsweise divergiert, wobei er nämlich in einem ersten Abschnitt 20 konstant ist und in einem zweiten Abschnitt 22 divergiert. Dabei ist ein definierter Ort 24 vorgesehen, an welchem die Elektroden 3, 5 einen kürzesten Abstand zueinander aufweisen und wo initial ein Entladungsfilament 13 gebildet wird.

Die Magnetfeldeinrichtung 15 weist hier einen Bandmagneten auf, der sich entlang der Anordnung der Elektroden 3, 5 erstreckt. Auch hier steht der Magnetfeldvektor B senkrecht auf der Bildebene von Figur 4. Die Funktionsweise dieses Ausführungsbeispiels der Vorrichtung 1 ist im Wesentlichen identisch zu der zuvor erläuterten Funktionsweise des dritten Ausführungsbeispiels gemäß Figur 3, wobei sich jedoch der relativ langgezogene, homogene Bereich zwischen den Elektroden 3, 5 mit konstantem Abstand ergibt, an den sich dann schließlich der zweite Abschnitt mit divergierenden Elektroden 3, 5 anschließt, wo schließlich auch der Abbruchbereich 19 ausgebildet ist. Der erste Abschnitt, in welchem die Elektroden 3, 5 zueinander einen konstanten Abstand aufweisen, kann vorzugsweise eine Länge von bis zu 100 cm aufweisen, insbesondere weil derzeit Bandmagnete erhältlich sind, welche jedenfalls aneinandergereiht eine solche Länge haben können. Dabei hängt die Länge des derart realisierbaren Bereichs insbesondere von der Länge zur Verfügung stehender Bandmagnete ab, wobei auch mehrere Bandmagnete hintereinander angeordnet werden können. Auf diese Weise sind prinzipiell der Länge des Entladungsbereichs 11 insgesamt keine grundsätzlichen Grenzen gesetzt. Praktische Grenzen ergeben sich einerseits aufgrund sinnvoller Abmessungen der Vorrichtung 1 und andererseits aufgrund gewünschter Frequenzen, mit welchen das Entladungsfilament 13 den Entladungsbereich 11 überstreicht, wobei diese Frequenzen insbesondere von der Magnetfeldstärke des Bandmagnets und andererseits von der Länge der Elektroden 3, 5 von dem Entstehungsort 24 des Entladungsfilaments 13 bis zu dem Abbruchbereich 19 abhängen. Statt eines Bandmagneten ist es auch möglich, dass eine Mehrzahl von Stabmagneten nebeneinander angeordnet sind, oder dass Elektromagneten, insbesondere Spulen, in entsprechender Weise nebeneinander entlang der Erstreckung der Elektroden 3, 5 angeordnet sind.

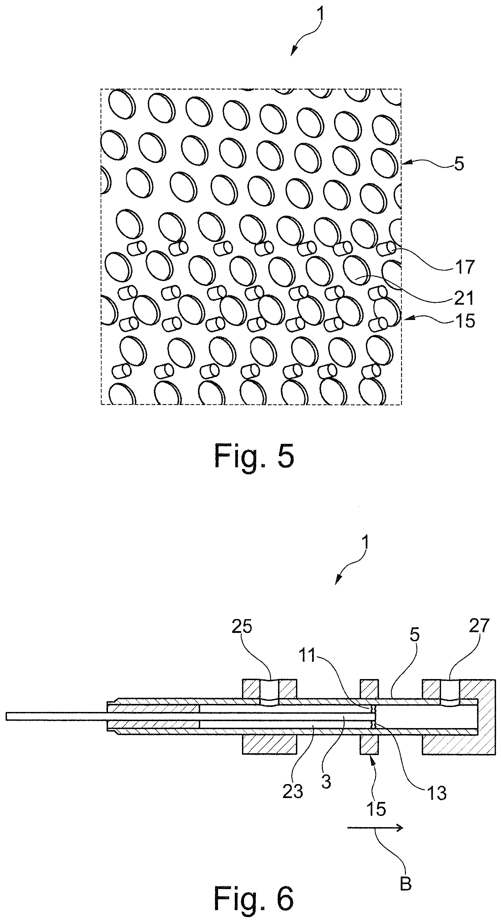

**Fig. 5** zeigt eine schematische Darstellung eines fünften Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Die zweite Elektrode 5 ist bei diesem Ausführungsbeispiel als flächige Elektrode mit einer Mehrzahl von Aussparungen 21 ausgebildet. Die Aussparungen 21 sind hier kreisförmig ausgebildet. Es sind aber auch andere, regelmäßig oder auch unregelmäßig umrandete Aussparungen, insbesondere rechteckförmige Aussparungen, oder Aussparungen, welche vorgegebene, ungleichmäßige Kantenverläufe aufweisen, beispielsweise nach Art des Verlaufs der Elektroden 3, 5 in den Ausführungsbeispielen gemäß den Figuren 3 und 4 - möglich.

Die erste Elektrode 3 ist hier nicht dargestellt. Bei dem konkret dargestellten Ausführungsbeispiel ist bevorzugt vorgesehen, dass in jeder Aussparung 21 eine draht- oder stabförmige Elektrode mittig angeordnet ist, wobei die Mehrzahl solcher Elektroden bevorzugt miteinander elektrisch verbunden und auf ein gemeinsames Potential gelegt werden können, welches sich von dem Potential der zweiten Elektrode 5 unterscheidet. Bei anderen Ausgestaltungen ist es auch möglich, dass sich die erste Elektrode 3 zumindest bereichsweise entlang wenigstens einer Kante wenigstens einer Aussparung 21 erstreckt, insbesondere parallel zu einer Oberfläche der flächigen Elektrode 5. Auch dabei ist es möglich, dass die erste Elektrode 3 als draht- oder stabförmige Elektrode ausgebildet ist.

Die Magnetfeldeinrichtung 15 weist hier eine Mehrzahl von Stabmagneten 17 auf, von denen der besseren Übersichtlichkeit wegen nur einer mit dem Bezugszeichen 17 gekennzeichnet ist. Diese Stabmagnete 17 sind um die Aussparungen 21 herum angeordnet und erzeugen vorzugsweise ein Magnetfeld, dessen Magnetfeldvektor senkrecht auf einer Oberfläche der zweiten Elektrode 5 steht. In den Aussparungen 21 gebildete Entladungsfilamente propagieren dann in den Aussparungen 21 in einer Weise, wie dies in Zusammenhang mit den Figuren 1 bis 4 erläutert wurde. Bei dem hier konkret dargestellten Ausführungsbeispiel würde sich insbesondere ein Verhalten wie bei dem Ausführungsbeispiel gemäß Figur 2 ergeben.

Anstelle der Stabmagnete 17 können auch Elektromagnete, insbesondere Spulen, vorgesehen sein, oder es können sowohl Elektromagnete als auch Stabmagnete 17 vorgesehen sein. Alternativ ist es auch möglich, dass jede Aussparung 21 oder jedenfalls eine Mehrzahl von Aussparungen 21 von - insbesondere jeweils - einem Ringmagneten umgriffen wird. Es ist auch ein gemeinsamer Ringmagnet oder eine gemeinsame Spule für eine Mehrzahl von Aussparungen 21 möglich.

Verschiedene, benachbarte Magnete und/oder Spulen können im Übrigen parallel oder antiparallel orientierte Magnetfelder aufweisen.

Die Aussparungen 21 sind im Betrieb der Vorrichtung 1 bevorzugt von einem Fluid durchströmt, welches dann mit dem in den Aussparungen 21 erzeugten Plasma behandelt wird.

Auch die Entladungsbereiche 11 der anderen Ausführungsbeispiele der Vorrichtung 1 gemäß den Figuren 1 bis 4 sind bevorzugt im Betrieb mit einem Fluid durchströmt, welches in den Entladungsbereichen 11 behandelt wird.

Werden - wie in Figur 5 dargestellt - kreisförmig umrandete Aussparungen 21 gewählt, ist es grundsätzlich möglich, ein Verhältnis von einer Gesamtfläche der Aussparungen 21 zu einer nicht ausgesparten Gesamtfläche der zweiten Elektrode 5 von 1:1 zu schaffen. Werden rechteckförmig berandete oder im Wesentlichen rechteckförmig berandete Aussparungen gewählt, die dann vorzugsweise mit Bandmagneten kombiniert werden, kann ein Verhältnis der insgesamt ausgesparten Fläche zu der insgesamt nicht ausgesparten Fläche der zweiten Elektrode 5 von 9:1 bereitgestellt werden, sodass vorteilhaft ein wesentlich reduzierter Strömungswiderstand für ein die zweite Elektrode 5 durchsetzendes Fluid geschaffen wird.

**Fig. 6** zeigt eine schematische Darstellung eines sechsten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Die Vorrichtung 1 ist hier als Strömungsrohr ausgebildet, in welchem die erste Elektrode 3 zentrisch, insbesondere mittig, als stabförmige Elektrode angeordnet ist, wobei die zweite Elektrode 5 als ringförmige Elektrode, hier als zylinderförmige Hohlelektrode, insbesondere als zylindrisches Rohrelement, ausgebildet ist. Dabei dient die zweite Elektrode 5 zugleich als Strömungs- oder Leitrohr für ein Fluid, welches im Betrieb der Vorrichtung 1 den Entladungsbereich 11 durchsetzt. Insoweit weist die Vorrichtung 1 hier einen Leitungsabschnitt 23 auf, der mit dem Entladungsbereich 11 in Fluidverbindung ist, sodass ein Fluid entlang des Leitungsabschnitts 23 durch den Entladungsbereich 11 geleitet werden kann. Dabei steht der Leitungsabschnitt 23 hier mit einer Fluidzuleitung 25 und einer Fluidableitung 27 in Fluidverbindung, wobei die Fluidzuleitung 25 und die Fluidableitung 27 insbesondere Verbindungselemente zu weiteren, hier nicht dargestellten Fluidleitungen, beispielsweise Verschraubungen zum Anschluss von Schläuchen, aufweisen können.

Die Magnetfeldeinrichtung 15 ist hier als Ringmagnet ausgebildet, welcher die Anordnung der Elektroden 3, 5 in dem Entladungsbereich 11 umgreift.

Die Funktionsweise des sechsten Ausführungsbeispiels gemäß Figur 6 ist dabei identisch zu der Funktionsweise, die mit Bezug auf das erste Ausführungsbeispiel gemäß Figur 1b) erläutert wurde, wobei eine entlang des kreisringförmigen Entladungsbereichs 11 propagierende Entladung erzeugt wird. Der Magnetfeldvektor B ist hier bevorzugt parallel zu der Längserstreckung der ersten Elektrode 3 gewählt, zeigt also in axialer Richtung, insbesondere in Längsrichtung der Vorrichtung 1.

Die propagierende Bewegung des Entladungsfilaments 13 entlang des Entladungspfads 11 und eine Strömungsgeschwindigkeit des Fluids durch den Entladungsbereich 11 werden so aufeinander abgestimmt, dass jedes den Entladungsbereich 11 durchströmende Volumenelement des Fluids wenigstens einmal von dem Entladungsfilament 13 überstrichen wird, sodass aus Sicht des strömenden Fluids quasi ein Entladungs- und insbesondere Plasmavorhang ausgebildet ist, durch welchen das Fluid strömt und mit dem das Fluid behandelt wird. Dabei wird das Fluid bevorzugt von Gerüchen, von Allergenen, von Mikroorganismen, insbesondere von Mikroben, Bakterien, Sporen, Pilzen, und/oder von Viren, oder von Partikeln, insbesondere von Nanopartikeln, bevorzugt von Schmutz oder Staub, gereinigt.

Das Fluid wird bevorzugt derart durch den Entladungsbereich 11 geleitet, dass in dem Entladungsbereich 11 Atmosphärendruck, oder ein Druck in der Nähe von Atmosphärendruck, vorzugsweise kein Grob- oder gar Fein- oder Hochvakuum, und auch kein Hochdruck, herrscht.

Als Fluid wird vorzugsweise Luft, Stickstoff, ein Edelgas, beispielsweise Argon, Kohlendioxid, ein Gasgemisch, oder ein Gemisch aus wenigstens einem Gas und wenigstens einer Flüssigkeit, beispielsweise Wasserdampf, verwendet.

Die Vorrichtung 1 kann in vorteilhafter Weise als Luftreinigungseinrichtung verwendet werden, insbesondere zur Luftreinigung in einer Wohnung, in einer Küche, insbesondere in einer Großküche, beispielsweise der Küche eines Gastronomiebetriebs, in einem Stall oder einer Fleischproduktionseinrichtung, in einer Chemieeinrichtung, in einem Büro, in einer Fabrik, in einer Kläranlage, in einer Mülldeponie, oder in einer anderen Einrichtung, in welcher es einer Reinigung von Luft von wenigstens einem der zuvor genannten Stoffe oder von weiteren Stoffen bedarf. Hierunter kann selbstverständlich auch eine medizinische Einrichtung, beispielsweise eine Arztpraxis oder ein Krankenhaus fallen.

Insgesamt zeigt sich, dass mit der Vorrichtung 1, mithilfe des Verfahrens und mittels der Verwendung der Vorrichtung 1 eine effektive Reinigung eines Fluids mit geringem Aufwand und kostengünstig möglich ist.

**Patentansprüche**

1. Vorrichtung (1) zur Erzeugung eines Plasmas, mit

- wenigstens einer ersten Elektrode (3),
- wenigstens einer von der ersten Elektrode (3) beabstandet angeordneten zweiten Elektrode (5),
- einer Spannungsquelle (7), die mit wenigstens einer Elektrode (3,5), ausgewählt aus der ersten Elektrode (3) und der zweiten Elektrode (5), derart verbunden ist, dass eine Potentialdifferenz zwischen der wenigstens einen ersten Elektrode (3) und der wenigstens einen zweiten Elektrode (5) durch die Spannungsquelle (7) erzeugbar ist, wobei
- die Spannungsquelle (7) als Gleichspannungsquelle ausgebildet ist, wobei
- die wenigstens eine erste Elektrode (3) und die wenigstens eine zweite Elektrode (5) wenigstens einen Entladungspfad (9) für eine elektrische Entladung in einem Entladungsbereich (11) zwischen der wenigstens einen ersten Elektrode (3) und der wenigstens einen zweiten Elektrode (5) definieren, mit
- einer Magnetfeldeinrichtung (15), die eingerichtet und relativ zu der wenigstens einen ersten Elektrode (3) und der wenigstens einen zweiten Elektrode (5) angeordnet ist, um in dem Entladungsbereich (11) ein Magnetfeld bereitzustellen, sodass
- ein Magnetfeldvektor (B) des Magnetfelds schräg zu dem Entladungspfad (9) orientiert ist, und mit
- einem Leitungsabschnitt (23), der mit dem Entladungsbereich (11) in Fluidverbindung ist, sodass ein Fluid entlang des Leitungsabschnitts (23) durch den Entladungsbereich (11) geleitet werden kann, wobei die Vorrichtung (1) eingerichtet ist,
- um das Fluid entlang des Leitungsabschnitts (23) durch den Entladungsbereich (11) zu leiten und
- zur Erzeugung eines nicht-thermischen Plasmas, wobei
- wenigstens ein Entladungsfilament (13) entlang des Entladungspfads (9) in dem Entladungsbereich (11) zwischen den wenigstens zwei Elektroden (3,5) erzeugt wird, wobei
- das Entladungsfilament (13) durch das Magnetfeld zu einer propagierenden Bewegung in dem Entladungsbereich (11) angetrieben wird,
- wobei im Betrieb der Vorrichtung (1) das Fluid durch den Entladungsbereich (11) geleitet wird, und **dadurch gekennzeichnet, dass**
- die Vorrichtung (1) so ausgestaltet ist, dass im Betrieb der Vorrichtung (1) die propagierende Bewegung des Entladungsfilaments (13) und eine Strömungsgeschwindigkeit des Fluids durch den Entladungsbereich (11) so aufeinander abgestimmt sind, dass jedes Volumenelement des den Entladungsbereich (11) durchströmenden Fluids, welches sich in einer Richtung senkrecht zu dem Entladungspfad (9) durch den Entladungsbereich (11) bewegt und eine in der Richtung senkrecht zu dem Entladungspfad (9) gemessene Dicke aufweist, die einer Dicke des Entladungsfilaments (13) entspricht, wenigstens einmal von dem Entladungsfilament (13) überstrichen wird.

2. Vorrichtung (1) nach Anspruch 1, **wobei**

a) die wenigstens eine erste Elektrode (3) als stab- oder drahtförmige Elektrode, und die wenigstens eine zweite Elektrode (5) als ringförmige Elektrode, welche die wenigstens eine erste Elektrode (3) - in Umfangsrichtung gesehen - umgreift; und/oder

b) die wenigstens eine erste Elektrode (3) und die wenigstens eine zweite Elektrode (5) als stab- oder drahtförmige Elektroden, und/oder

c) die wenigstens eine zweite Elektrode (5) als flächige Elektrode mit einer Mehrzahl von Aussparungen (21) ausgebildet ist/sind.

**3.** Vorrichtung (1) nach einem der vorhergehenden Ansprüche, **wobei** ein Abstand zwischen der wenigstens einen ersten Elektrode (3) und der wenigstens einen zweiten Elektrode (5) entlang einer Erstreckung wenigstens einer der Elektroden (3,5)

a) konstant ist, oder
b) divergiert, oder
c) in einem ersten Abschnitt konstant ist und in einem zweiten Abschnitt divergiert.

**4.** Vorrichtung (1) nach einem der vorhergehenden Ansprüche, **wobei** die Magnetfeldeinrichtung (15) wenigstens einen Permanentmagnet und/oder wenigstens einen Elektromagnet aufweist.

**5.** Vorrichtung (1) nach einem der vorhergehenden Ansprüche, **wobei** die Magnetfeldeinrichtung (15)

a) wenigstens einen Ringmagnet oder wenigstens eine Spule aufweist, und/oder
b) eine Mehrzahl von Stabmagneten (17) oder Spulen aufweist, welche entlang zumindest einer Elektrode, ausgewählt aus der ersten Elektrode (3) und der zweiten Elektrode (5), angeordnet ist, und/oder
c) wenigstens einen Bandmagnet aufweist, welcher entlang zumindest einer Elektrode (3,5), ausgewählt aus der wenigstens einen ersten Elektrode (3) und der wenigstens einen zweiten Elektrode (5), angeordnet ist.

**6.** Verfahren zum Erzeugen eines Plasmas mittels einer Vorrichtung (1) nach einem der Ansprüche 1 bis 5, **wobei**

- das wenigstens eine Entladungsfilament (13) entlang des Entladungspfads (9) in dem Entladungsbereich (11) zwischen den wenigstens zwei Elektroden (3,5) erzeugt wird, wobei
- das Magnetfeld bereitgestellt wird, welches den Entladungsbereich (11) schräg zu dem Entladungspfad (9) durchsetzt, und wobei
- das Entladungsfilament (13) durch das Magnetfeld zu der propagierenden Bewegung in dem Entladungsbereich (11) angetrieben wird,
- wobei die Vorrichtung (1) ein nicht-thermisches Plasma erzeugt, und wobei
- das Fluid durch den Entladungsbereich (11) geleitet wird,
**dadurch gekennzeichnet, dass**
- die propagierende Bewegung des Entladungsfilaments (13) und die Strömungsgeschwindigkeit des Fluids durch den Entladungsbereich (11) so aufeinander abgestimmt werden, dass jedes Volumenelement des den Entladungsbereich (11) durchströmenden Fluids, welches sich in einer Richtung senkrecht zu dem Entladungspfad (9) durch den Entladungsbereich (11) bewegt und eine in der Richtung senkrecht zu dem Entladungspfad (9) gemessene Dicke aufweist, die einer Dicke des Entladungsfilaments (13) entspricht, wenigstens einmal von dem Entladungsfilament (13) überstrichen wird.

**7.** Verfahren nach Anspruch 6, **wobei** in dem Entladungsbereich (11) Atmosphärendruck herrscht.

**8.** Verfahren nach einem der Ansprüche 6 oder 7, **wobei** als Fluid Luft, Stickstoff, Edelgas, insbesondere Argon, Kohlendioxid, ein Gasgemisch, oder ein Gemisch aus wenigstens einem Gas und wenigstens einer Flüssigkeit, insbesondere Wasserdampf, verwendet wird.

**9.** Verfahren nach einem der Ansprüche 6 bis 8, **wobei** das den Entladungsbereich (11) durchströmende Fluid

a) von Gerüchen,
b) von Allergenen,
c) von Mikroorganismen, und/oder
d) von Partikeln
gereinigt wird.

**10.** Verwendung einer Vorrichtung (1) nach einem der Ansprüche 1 bis 5,

a) als Luftreinigungseinrichtung in

- einer Wohnung,
- einer Küche, insbesondere eines Gastronomiebetriebs,
- einem Stall oder einer Fleischproduktionseinrichtung,
- einer Chemieeinrichtung,
- einem Büro,
- einer Fabrik,
- einer Kläranlage,
- einer Mülldeponie, oder
- einer medizinischen Einrichtung, und/oder

b) zur Reinigung eines Fluids von

- Gerüchen,
- Allergenen,
- Mikroorganismen, und/oder
- Partikeln.

**Claims**

1. Apparatus (1) for generating a plasma, comprising

- at least one first electrode (3),
- at least one second electrode (5) located at a distance from the first electrode (3),
- a voltage source (7) which is connected in such a manner to at least one electrode (3, 5), selected from the first electrode (3) and the second electrode (5), that a potential difference between the at least one first electrode (3) and the at least one second electrode (5) can be produced by the voltage source (7), wherein
- the voltage source (7) is configured as a DC voltage source, wherein
- the at least one first electrode (3) and the at least one second electrode (5) define at least one discharge path (9) for an electrical discharge in a discharge region (11) between the at least one first electrode (3) and the at least one second electrode (5), having
- a magnetic field device (15) adapted and located relative to the at least one first electrode (3) and the at least one second electrode (5) to provide a magnetic field in the discharge region (11) such that
- a magnetic field vector (B) of the magnetic field is oriented at an angle to the discharge path (9), and comprising
- a conduit portion (23) in fluid communication with the discharge region (11) so that a fluid can be conveyed along the conduit portion (23) through the discharge region (11), wherein
the apparatus (1) is adapted,
- to convey the fluid along the conduit portion (23) through the discharge region (11), and
- for generating a non-thermal plasma, wherein
- at least one discharge filament (13) is generated along the discharge path (9) in the discharge region (11) between the at least two electrodes (3, 5), wherein
- the discharge filament (13) is driven by the magnetic field to a propagating motion in the discharge region (11),
- wherein, in operation of the apparatus, the fluid is conveyed through the discharge region (11), and
**characterized in that**
- the apparatus (1) is configured such that, in operation of the apparatus (1), the propagating motion of the discharge filament (13) and a flow rate of the fluid through the discharge region (11) are adjusted to each other such that each volume element of the fluid flowing through the discharge region (11), which moves in a direction perpendicular to the discharge path (9) through the discharge region (11) and has a thickness, measured in the direction perpendicular to the discharge path (9), corresponding to a thickness of the discharge filament (13), is overrun at least once by the discharge filament (13).

2. Apparatus (1) according to claim 1, **wherein**

a) the at least one first electrode (3) is formed as a rod-shaped or wire-shaped electrode, and the at least one second electrode (5) is formed as an annular electrode, which surrounds the at least one first electrode (3) - seen in the circumferential direction; and/or
b) the at least one first electrode (3) and the at least one second electrode (5) are formed as rod-shaped or wire-shaped electrodes, and/or

c) the at least one second electrode (5) is formed as a flat electrode with a plurality of recesses (21).

3. Apparatus (1) according to one of the preceding claims, **wherein** a distance between the at least one first electrode (3) and the at least one second electrode (5) along an extension of at least one of the electrodes (3, 5) is

   a) constant, or
   b) divergent, or
   c) constant in a first section and diverges in a second section.

4. Apparatus (1) according to any one of the preceding claims, **wherein** the magnetic field device (15) comprises at least one permanent magnet and/or at least one electromagnet.

5. Apparatus (1) according to any one of the preceding claims, **wherein** the magnetic field device (15) comprises

   a) at least one ring magnet or at least one coil, and/or
   b) a plurality of bar magnets (17) or coils located along at least one electrode selected from the first electrode (3) and the second electrode (5), and/or
   c) at least one band magnet located along at least one electrode (3, 5) selected from the at least one first electrode (3) and the at least one second electrode (5).

6. Method for generating a plasma by means of an apparatus (1) according to any one of claims 1 to 5, **wherein**

   - the at least one discharge filament (13) is generated along the discharge path (9) in the discharge region (11) between the at least two electrodes (3, 5), wherein
   - the magnetic field is provided which passes through the discharge region (11) at an angle to the discharge path (9), and wherein
   - the discharge filament (13) is driven by the magnetic field to a propagating motion in the discharge region (11),
   - wherein the apparatus (1) generates a non-thermal plasma, and wherein
   - the fluid is conveyed through the discharge region (11),
   **characterized in that**
   - the propagating motion of the discharge filament (13) and the flow rate of the fluid through the discharge region (11) are adjusted to each other such that each volume element of the fluid flowing through the discharge region (11), which moves in a direction perpendicular to the discharge path (9) through the discharge region (11) and has a thickness, measured in the direction perpendicular to the discharge path (9), corresponding to a thickness of the discharge filament (13), is overrun at least once by the discharge filament (13).

7. Method according to claim 6, **wherein** atmospheric pressure prevails in the discharge region (11).

8. Method according to one of claims 6 or 7, **wherein** air, nitrogen, noble gas, in particular argon, carbon dioxide, a gas mixture, or a mixture of at least one gas and at least one liquid, in particular water vapor, is used as fluid.

9. Method according to any one of claims 6 to 8, **wherein** the fluid flowing through the discharge region (11) is cleaned

   a) of odors,
   b) of allergens,
   c) of microorganisms, and/or
   d) of particles.

10. Use of an apparatus (1) according to any one of claims 1 to 5,

   a) as an air purification device in

      - an apartment,
      - a kitchen, in particular of a gastronomic establishment,
      - a stable or a meat production facility,
      - a chemical facility,
      - an office
      - a factory

- a sewage treatment plant,
- a landfill, or
- a medical facility, and/or

b) for the purification of a fluid of

- odors,
- allergens,
- microorganisms, and/or
- particles.

**Revendications**

1. Dispositif (1) pour la génération d'un plasma, comprenant

- au moins une première électrode (3),
- au moins une deuxième électrode (5) disposée à distance de la première électrode (3),
- une source de tension (7), qui est reliée à au moins une électrode (3, 5), sélectionnée parmi la première électrode (3) et la deuxième électrode (5), de telle sorte qu'une différence de potentiel entre l'au moins une première électrode (3) et l'au moins une deuxième électrode (5) puisse être générée par la source de tension (7), dans laquelle
- la source de tension (7) est réalisée sous forme de source de tension continue, dans laquelle
- l'au moins une première électrode (3) et l'au moins une deuxième électrode (5) définissent au moins un trajet de décharge (9) pour une décharge électrique dans une zone de décharge (11) entre l'au moins une première électrode (3) et l'au moins une deuxième électrode (5), avec
- un dispositif de champ magnétique (15) conçu et disposé relativement à l'au moins une première électrode (3) et à l'au moins une deuxième électrode (5) pour fournir un champ magnétique dans la zone de décharge (11), de sorte que
- un vecteur de champ magnétique (B) du champ magnétique est orienté obliquement par rapport au trajet de décharge (9), et avec
- une section de conduit (23) qui est en connexion fluidique avec la zone de décharge (11), de sorte qu'un fluide peut être dirigé le long de la section de conduit (23) à travers la zone de décharge (11), dans laquelle le dispositif (1) est conçu pour
- diriger le fluide le long de la section de conduite (23) à travers la zone de décharge (11) et
- générer un plasma non thermique, dans lequel
- au moins un filament de décharge (13) est généré le long du trajet de décharge (9) dans la zone de décharge (11) entre les au moins deux électrodes (3, 5), dans lequel
- le filament de décharge (13) est entraîné par le champ magnétique dans un mouvement de propagation dans la zone de décharge (11),
- le fluide étant dirigé à travers la zone de décharge (11) pendant le fonctionnement du dispositif (1), et **caractérisé en ce que**
- le dispositif (1) est conçu de telle sorte que, pendant le fonctionnement du dispositif (1), le mouvement de propagation du filament de décharge (13) et une vitesse d'écoulement du fluide à travers la zone de décharge (11) sont accordés l'un à l'autre de telle sorte que chaque élément de volume du fluide traversant la zone de décharge (11), qui se déplace dans une direction perpendiculaire au trajet de décharge (9) à travers la zone de décharge (11) et qui présente une épaisseur, mesurée dans la direction perpendiculaire au trajet de décharge (9), qui correspond à une épaisseur du filament de décharge (13), est recouvert au moins une fois par le filament de décharge (13).

2. Dispositif (1) selon la revendication 1, **dans lequel**

a) l'au moins une première électrode (3) est réalisée comme une électrode en forme de tige ou de fil, et l'au moins une deuxième électrode (5) est réalisée comme une électrode annulaire qui entoure l'au moins une première électrode (3) - vue dans le sens périphérique ; et/ou
b) l'au moins une première électrode (3) et l'au moins une deuxième électrode (5) sont réalisées comme électrodes en forme de tige ou de fil, et/ou
c) l'au moins une deuxième électrode (5) est réalisée comme une électrode plane avec une pluralité d'évidements (21).

3. Dispositif (1) selon l'une des revendications précédentes, **dans lequel** une distance entre l'au moins une première électrode (3) et l'au moins une deuxième électrode (5) le long d'une extension d'au moins une des électrodes (3, 5)

    a) est constant, ou
    b) diverge, ou
    c) est constante dans une première section et diverge dans une deuxième section.

4. Dispositif (1) selon l'une des revendications précédentes, **dans lequel** le dispositif de champ magnétique (15) présente au moins un aimant permanent et/ou au moins un électroaimant.

5. Dispositif (1) selon l'une des revendications précédentes, **dans lequel** le dispositif de champ magnétique (15)

    a) présente au moins un aimant annulaire ou au moins une bobine, et/ou
    b) présente une pluralité des aimants en forme de tige (17) ou de bobines disposés le long d'au moins une électrode sélectionnée parmi la première électrode (3) et la seconde électrode (5), et/ou
    c) présente au moins un aimant en bande disposé le long d'au moins une électrode (3, 5) sélectionnée parmi l'au moins une première électrode (3) et l'au moins une deuxième électrode (5).

6. Procédé de génération d'un plasma au moyen d'un dispositif (1) selon l'une des revendications 1 à 5, **dans lequel**

    - le au moins un filament de décharge (13) est généré le long du trajet de décharge (9) dans la zone de décharge (11) entre les au moins deux électrodes (3, 5), dans lequel
    - ce champ magnétique est fourni, lequel traverse la zone de décharge (11) de manière oblique par rapport au trajet de décharge (9), et dans lequel
    - le filament de décharge (13) est entraîné par le champ magnétique dans un mouvement de propagation dans la zone de décharge (11),
    - dans lequel le dispositif (1) génère un plasma non thermique, et dans lequel
    - le fluide est dirigé à travers la zone de décharge (11),
    **caractérisé en ce que**
    - le mouvement de propagation du filament de décharge (13) et la vitesse d'écoulement du fluide à travers la zone de décharge (11) sont accordés l'un à l'autre de telle sorte que chaque élément de volume du fluide traversant la zone de décharge (11), qui se déplace à travers la zone de décharge (11) dans une direction perpendiculaire au trajet de décharge (9) et qui présente une épaisseur, mesurée dans la direction perpendiculaire au trajet de décharge (9), qui correspond à une épaisseur du filament de décharge (13), est recouvert au moins une fois par le filament de décharge (13).

7. Procédé selon la revendication 6, **dans lequel** la pression atmosphérique prévaut dans la zone de décharge (11).

8. Procédé selon l'une des revendications 6 ou 7, **dans lequel** on utilise comme fluide de l'air, de l'azote, un gaz rare, notamment de l'argon, du dioxyde de carbone, un mélange de gaz, ou un mélange d'au moins un gaz et d'au moins un liquide, notamment de la vapeur d'eau.

9. Procédé selon l'une des revendications 6 à 8, **dans lequel** le fluide traversant la zone de décharge (11) est purifié

    a) d'odeurs,
    b) d'allergènes,
    c) de micro-organismes, et/ou
    d) de particules.

10. Utilisation d'un dispositif (1) selon l'une des revendications 1 à 5,

    a) comme dispositif de purification de l'air dans

        - une habitation,
        - une cuisine, notamment d'un établissement de restauration,
        - une étable ou d'une installation de production de viande,

- un établissement chimique,
- un bureau,
- une usine,
- une station d'épuration des eaux,
- une décharge de déchets, ou
- un établissement médical, et/ou

b) pour purifier un fluide

- des odeurs,
- d'allergènes,
- des micro-organismes, et/ou
- des particules.

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070120495 A1